# EUROPEAN PATENT APPLICATION

(11) **EP 2 221 299 A2**
(43) Date of publication of application: **25.08.2010**
(21) Application number: 10250220.0
(22) Date of filing: 09.02.2010
(51) Int. Cl.: C07D 237/32, C07D 487/04

(54) **Preparation of cilazapril intermediates**

(30) Priority: 11.02.2009 IN CH02912009; 09.06.2009 US 185242 P
(71) Applicant: Dr. Reddy's Laboratories Ltd., Hyderabad 500 016, Andhra Pradesh (IN)
(72) Inventor: Elias, Sergio Arturo Ferrino, 62738 Yautepec Morelos (MX); Kunhimon, Syam Kumar Unniaranpurakkal, Thrissur, Kerala (IN); More, Satish Sukhlal, Navsari 396445, Gujarat (IN); Tovar, Eduardo, 62578 Civac, Jiutepec Morelos (MX); Dahanukar, Vilas H, Hyderabad-500008, Andhrapradesh (IN); Meruva, Suresh Babu, Kadapa, Andhra Pradesh 516162 (IN); Akula, Raghunadh, Srikakulam, Andhra Pradesh 532222 (IN); Nuka, Anil Kumar, Kukatpally, Hyderabad 500072 (IN); Puppala, Jyothi, Hyderabad, Andhra Pradesh 500073 (IN); Gudla, Santosh, Adilabad, Andhra Pradesh 504301 (IN)
(74) Representative: Bates, Philip Ian

(57) **Abstract**

The present application relates to cilazapril intermediates and processes for the preparation of cilazapril intermediates, including (S)-6,11-dioxo-1,2,3,4,6,11-hexahydro-pyridazino[1,2-b]naphthalazine-1-carboxylic acid and its salts.

## Description

### INTRODUCTION

In aspects, the invention relates to cilazapril intermediates and processes for the preparation of cilazapril intermediates, including (S)-6,11-dioxo-1,2,3,4,6,11-hexahydro-pyridazino[1,2-b]naphthalazine-1-carboxylic acid and its salts.

Cilazapril has a chemical name (1S,9S)-9-[[(2S)-1-ethoxy-1-oxo-4-phenylbutan-2-yl]amino]-10-oxo-1,2,3,4,6,7,8,9-octahydropyridazino[1,2-a]diazepine-1-carboxylic acid, and has structural Formula I.

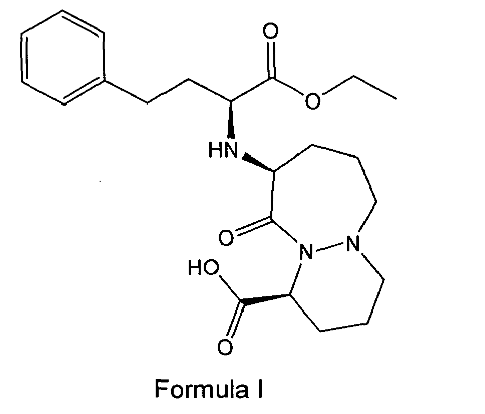

Cilazapril is an ACE inhibitor useful as an anti-hypertensive agent.

U.S. Patent No. 4,512,924 describes cilazapril generically, processes for its preparation, and its use as a medicament.

U.S. Patent No. 5,750,690 describes a process for preparation of the intermediate (S)-6,11-dioxo-1,2,3,4,6,11-hexahydro-pyridazino[1,2-b]naphthalazine-1-carboxylic acid, which process involves the hydrogenation of 3,4,6,11-tetrahydro-6-11-dioxo-pyridao[1,2-b]phthalazine-1-carboxylic acid and optically active ruthenium-diphosphine complex in an autoclave.

International Application Publication No. WO 01/094351 describes a process for preparing (S)-6,11-dioxo-1,2,3,4,6,11-hexahydro-pyridazino[1,2-b]naphthalazine-1-carboxylic acid that involves reacting (S)-hexahydro-pyridazine-3-carboxylic acid methyl ester hydrochloride, phthalic anhydride, and diisopropylethylamine in toluene, to obtain (S)-6,11-dioxo-1,2,3,4-tetrahydropyridazino[1,2-b]phthalazine-1-carboxylic acid methyl ester. This ester is hydrolyzed to the corresponding acid with KOH in methanol.

European Patent Application No. 667350 describes the preparation of the intermediate (S)-6,11-dioxo-1,2,3,4,6,11-hexahydro-pyridazino[1,2-b]naphthalazine-1-carboxylic acid, which preparation involves hydrogenation of 3,4,6,11-tetrahydro-6-11-dioxo-pyridao[1,2-b]phthalazine-1-carboxylic acid triethylammonium salt or sodium salt using ((S)-MeOBIPHEP-Ts-N(a)Ru(OCOF₃)₂ as a catalyst.

Rodriguez et al. in Synthesis, 1988, 7, 534-5 describe a process in which α,β-unsaturated aldehydes are reacted with monoalkyl malonate and pyridine with a catalytic amount of DMAP in a regio- and stereo-selective process, to yield 2,4-pentadienoic esters with 2E-stereochemistry.

### SUMMARY

Aspects of the invention include processes for the preparation of 6,11-dioxo-1,2,3,4,6,11-hexahydro-pyridazino[1,2-b]naphthalazine-1-carboxylic acid, and its esters and salts, embodiments comprising at least one of the steps of:
(a) reacting a monoalkyl malonic acid of Formula II or a salt thereof with acrolein of Formula III, in the presence of a pyridine reagent, to obtain a 2,4-pentadienylalkyl ester of Formula IV, 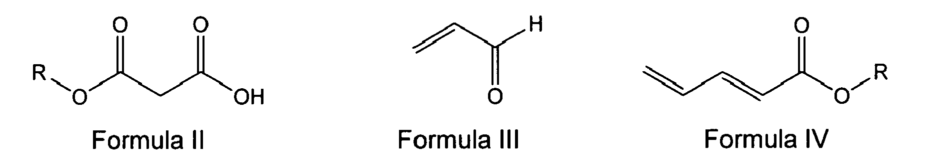 wherein R is C₁₋₆ alkyl, benzyl, or phenyl;
(b) reacting a 2,4-pentadienylalkyl ester of Formula IV with phthalizide of Formula V, in the presence of an oxidizing agent, to give a 6,11-dioxo-1,4,6,11-tetrahydro-pyridazino[1,2-b]phthalazine-1-carboxylic acid alkyl ester of Formula VI, 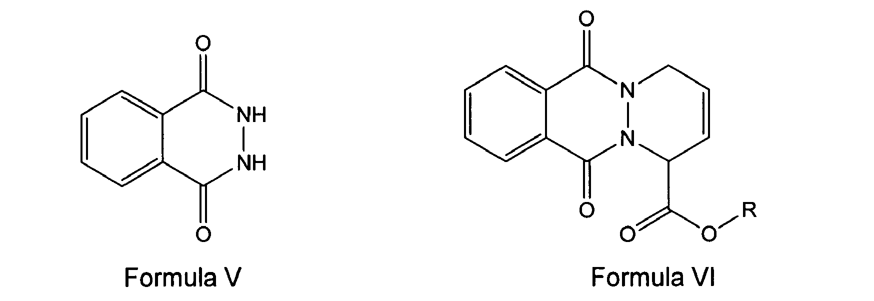 wherein R is as defined hereinabove;
(c) reducing a 6,11-dioxo-1,4,6,11-tetrahydro-pyridazino[1,2-b]phthalazine-1-carboxylic acid alkyl ester of Formula VI, to obtain an ester intermediate of Formula VII; and
(d) optionally, hydrolyzing an ester intermediate of Formula VII to give 6,11-dioxo-1,2,3,4,6,11-hexahydro-pyridazino[1,2-b]naphthalazine-1-carboxylic acid of Formula VIII, 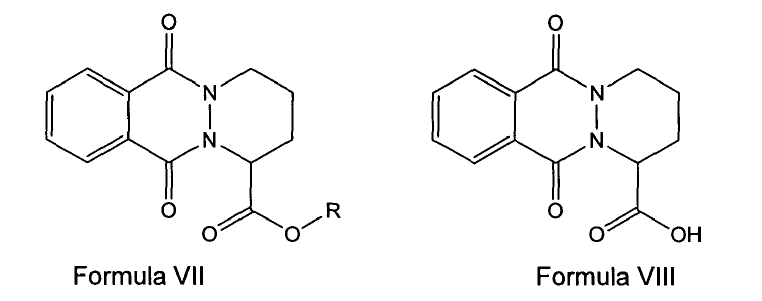
wherein R is as defined hereinabove.

Aspects of the invention include the compound (S)-6,11-dioxo-1,2,3,4,6,11-hexahydro-pyridazino[1,2-b]naphthalazine-1-carboxylic acid of Formula IX, 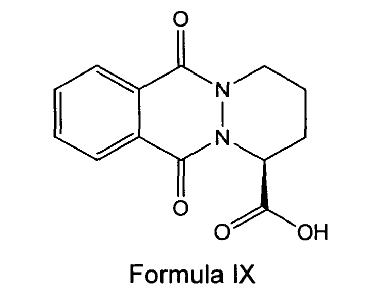 or a salt thereof, and processes for its preparation, embodiments comprising at least one of the steps of:
(a) reacting 6,11-dioxo-1,2,3,4,6,11-hexahydro-pyridazino[1,2-b]naphthalazine-1-carboxylic acid of Formula VIII with an optically pure amine to form a diastereomeric salt of Formula X, 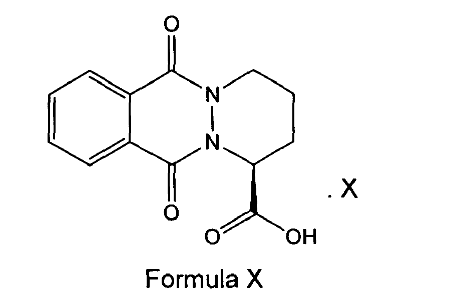 wherein X is an optically pure amine; and
(b) converting a diastereomeric salt of Formula X into (S)-6,11-dioxo-1,2,3,4,6,11-hexahydro-pyridazino[1,2-b]naphthalazine-1-carboxylic acid or a salt thereof.

Aspects of the invention include optically pure N-alkyl-D-glucamine salts of 6,11-dioxo-1,2,3,4,6,11-hexahydro-pyridazino[1,2-b]naphthalazine-1-carboxylic acid.

Aspects of the invention include processes for racemizing (R)-6,11-dioxo-1,2,3,4,6,11-hexahydro-pyridazino[1,2-b]naphthalazine-1-carboxylic acid comprising the step of reacting (R)-6,11-dioxo-1,2,3,4,6,11-hexahydro-pyridazino[1,2-b]naphthalazine-1-carboxylic acid with a suitable reagent, to obtain 6,11-dioxo-1,2,3,4,6,11-hexahydro-pyridazino[1,2-b]naphthalazine-1-carboxylic acid.

### DETAILED DESCRIPTION

All percentages and ratios used herein are by weight of the total composition and all measurements made are at about 25°C and about normal pressure unless otherwise designated. All temperatures are in degrees Celsius unless specified otherwise. As used herein, "comprising" means the elements recited, or their equivalent in structure or function, plus any other element or elements that are not recited. The terms "having" and "including" are also to be construed as open ended. All ranges recited herein include the endpoints, including those that recite a range "between" two values. The terms "about," "generally," "substantially,", and the like, are to be construed as modifying another term or value such that it is not an absolute, as defined by the circumstances and context as understood by those of skiii in the art. This includes, at very least, the degree of expected experimental error, technique error, and instrument error for a given technique used to measure a value. Whether so indicated or not, all values recited herein are approximate.

Aspects of the invention include processes for the preparation of 6,11-dioxo-1,2,3,4,6,11-hexahydro-pyridazino[1,2-b]naphthalazine-1-carboxylic acid, and its esters and salts, embodiments comprising at least one of the steps of:
(a) reacting a monoalkyl malonic acid of Formula II or a salt thereof with acrolein of Formula III, in the presence of a pyridine reagent, to obtain a 2,4-pentadienylalkyl ester of Formula IV, 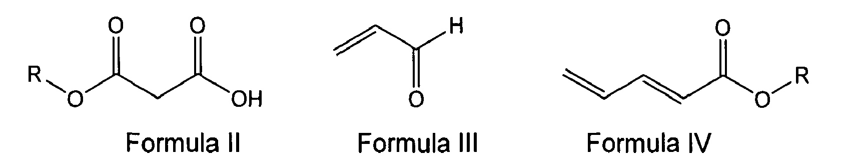 wherein R is C₁₋₆ alkyl, benzyl, or phenyl;
(b) reacting a 2,4-pentadienylalkyl ester of Formula IV with phthalizide of Formula V, in the presence of an oxidizing agent, to give a 6,11-dioxo-1,4,6,11-tetrahydro-pyridazino[1,2-b]phthalazine-1-carboxylic acid alkyl ester of Formula VI, 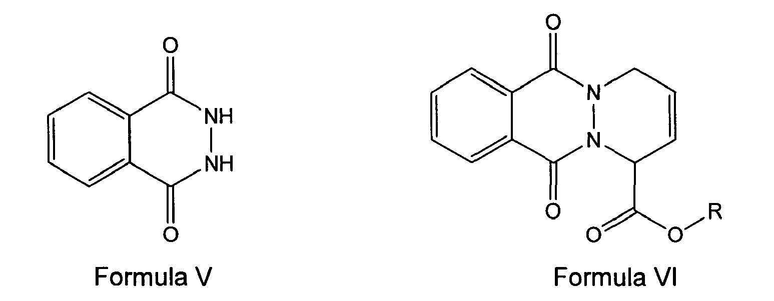 wherein R is as defined hereinabove;
(c) reducing a 6,11-dioxo-1,4,6,11-tetrahydro-pyridazino[1,2-b]phthalazine-1-carboxylic acid alkyl ester of Formula VI, to obtain an ester intermediate of Formula VII; and
(d) optionally, hydrolyzing an ester intermediate of Formula VII to give 6,11-dioxo-1,2,3,4,6,11-hexahydro-pyridazino[1,2-b]naphthalazine-1-carboxylic acid of Formula VIII, 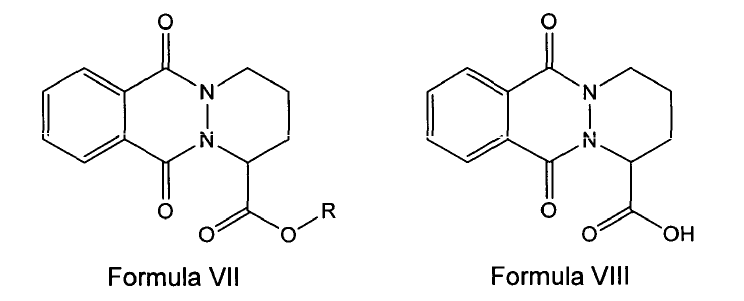
wherein R is as defined hereinabove.

Step (a) involves reacting a monoalkyl malonic acid of Formula II or a salt thereof with acrolein of Formula III, in the presence of a pyridine reagent, to obtain a 2,4-pentadienylalkyl ester of Formula IV.

The monoalkyl malonic acid of Formula II that is used in the processes may be obtained from commercial sources, or it may be prepared, for example, by reacting dialkyl malonate with a base in an alcohol solvent, to give monoalkylmaolnic acid or a salt thereof. Suitable bases that may be used in the reaction include, but are not limited to, sodium hydroxide (NaOH), potassium hydroxide (KOH), and the like. Suitable alcohol solvents that may be used in the reaction include, but are not limited to, methanol, ethanol, isopropanol, n-butanol, and the like. For example, a potassium salt of monomethyl malonate can be used in the reaction of step (a) because it is relatively more stable and suitable for commercial manufacturing when compared to monomethyl malonate. It is prepared by reacting diethyl malonate with KOH in methanol.

For example, an alkali salt of a monoalkyl malonic acid is reacted with an anhydrous acid source to obtain a corresponding monoalkyl malonic acid, which is then reacted *in situ* with the pyridine reagent.

Anhydrous acid sources that may be used include, but are not limited to, anhydrous acids, such as, for example, acetic acid, formic acid, sulfuric acid, hydrochloric acid, alkylsulfonic acids, arylsulfonic acid, and their salts with amines. For example, the anhydrous acid source may be pyridine hydrochloride or pyridine sulphate.

Suitable pyridine reagents that may be used include, but are not limited to, pyridine, dimethylaminopyridine (DMAP), 2,6-dialkylpyridines, and the like.

Suitable solvents that may be used in the reaction of step (a) include, but are not limited to: N,N-dimethylformamide (DMF); tetrahydrofuran (THF); hydrocarbons, such as, for example, toluene and xylene; halogenated hydrocarbons, such as, for example, dichloromethane, dichloroethane, chloroform, chlorobenzene, and the like; and any mixtures thereof.

Acrolein may be added slowly to the reaction mixture over a period of about 1 to 4 hours, or longer, in order to minimize the formation of impurities.

Suitable temperatures for conducting the reaction may range from about 20°C to about 80°C, or from about 40°C to about 50°C, and the reaction may proceed for a period of about 40-48 hours, or longer, for complete conversion of the reagents.

After completion, the reaction may be quenched with water and the product may be extracted into a water immiscible organic solvent. The organic layer may be distilled completely to obtain the product as a residue or it may be directly used in the next reaction step.

Step (b) involves reacting a 2,4-pentadienylalkyl ester of Formula IV with phthalizide of Formula V, in the presence of an oxidizing agent, to give a 6,11-dioxo-1,4,6,11-tetrahydro-pyridazino[1,2-b]phthalazine-1-carboxylic acid alkyl ester of Formula VI.

Suitable oxidizing agents include, but are not limited to sodium hypochlorite, N-bromosuccinimide, dibromohydantoin, and the like.

The reaction may be carried out in the presence of an acid, such as, for example, hydrochloric acid, hydrobromic acid, sulphuric acid, phosphoric acid, acetic acid, and the like.

Suitable solvents for the reaction include, but are not limited to: halogenated hydrocarbons, such as, for example, dichloromethane, 1,2-dichloroethane, chloroform, and the like; ketones, such as, for example, acetone, ethyl methyl ketone, methyl isobutyl ketone, and the like; esters, such as, for example, ethyl acetate, n-propyl acetate, t-butyl acetate, and the like; ethers, such as, for example, diethyl ether, dimethyl ether, diisopropyl ether, methyl tertiary-butyl ether, tetrahydrofuran, 1,4-dioxane, and the like; hydrocarbons, such as, for example, toluene, xylene, and the like; nitriles, such as, for example, acetonitrile, propionitrile, and the like; water; and any mixtures thereof.

The oxidizing reagent may be added to the reaction mixture slowly, such as over a period of about 5 to 10 hours, below 10°C. The reaction may be maintained for 1-5 hours below 10°C for completion of the reaction and then quenched with an aqueous sodium bisulfite solution.

The reaction mixture may be neutralized with an aqueous base and then extracted with an organic solvent. The organic layer containing the product may be used for the next reaction step directly or it can be distilled to obtain a residue.

The residue obtained may be further purified by crystallization from a suitable solvent, such as, for example, acetone, methyl ethyl ketone, methyl isobutyl ketone, methanol, ethanol, isopropanol, ethyl acetate and the like.

Step (c) involves reducing a 6,11-dioxo-1,4,6,11-tetrahydro-pyridazino[1,2-b]phthalazine-1-carboxylic acid alkyl ester of Formula VI, to obtain an ester intermediate of Formula VII.

Suitable reducing agents that may be used in the reaction of step (c) include, but are not limited to, metal catalysts, such as, for example, nickel, platinum, palladium, iridium, ruthenium, and the like, in combination with hydrogen or sources of hydrogen such as formic acid and its derivatives like ammonium formate.

Suitable solvents that may be used in the reaction of step (c) include, but are not limited to: C₁₋₆ straight chain or branched alcohols, such as, for example, methanol, ethanol, isopropanol, butanol, and the like; hydrocarbons, such as, for example, toluene, xylene, and the like; and any mixtures thereof.

After completion of the reaction, the catalyst may be removed by filtration. The organic layer containing the product may be distilled to obtain the product as a residue, or it may be used in the next reaction step directly.

Step (d) involves optionally hydrolyzing an ester intermediate of Formula VII, to give 6,11-dioxo-1,2,3,4,6,11-hexahydro-pyridazino[1,2-b]naphthalazine-1-carboxylic acid of Formula VIII.

For example, 6,11-dioxo-1,2,3,4,6,11-hexahydro-pyridazino[1,2-b]phthalazine-1-carboxylic acid methyl ester of Formula VII may be hydrolyzed in the presence of a base to obtain the corresponding acid.

Suitable bases include, but are not limited to, sodium hydroxide, potassium hydroxide, lithium hydroxide, and the like.

Suitable solvents that may be used in the reaction include, but are not limited to: water; C₁₋₆ straight chain or branched alcohols, such as, for example, methanol, ethanol, isopropanol, butanol, and the like; and any mixtures thereof.

Aspects of the invention include (S)-6,11-dioxo-1,2,3,4,6,11-hexahydro-pyridazino[1,2-b]naphthalazine-1-carboxylic acid of Formula IX or a salt thereof and processes for its preparation, embodiments comprising at least one of the steps of: 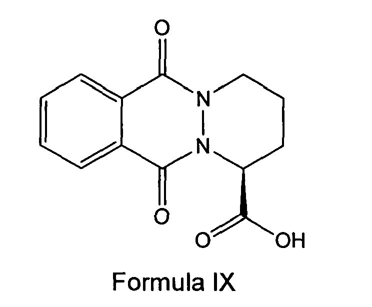
(a) reacting 6,11-dioxo-1,2,3,4,6,11-hexahydro-pyridazino[1,2-b]naphthalazine-1-carboxylic acid of Formula VIII with an optically pure amine, to form a diastereomeric salt of Formula X, wherein X is an optically pure amine; and
(b) converting a diastereomeric salt of Formula X into (S)-6,11-dioxo-1,2,3,4,6,11-hexahydro-pyridazino[1,2-b]naphthalazine-1-carboxylic acid or a salt thereof.

Step (a) involves reacting 6,11-dioxo-1,2,3,4,6,11-hexahydro-pyridazino[1,2-b]naphthalazine-1-carboxylic acid of Formula VIII with an optically pure amine to form a diastereomeric salt of Formula X, wherein X is an optically pure amine.

"Optically pure" means that the chiral purity of the compound is more than about 95%, or more than about 99%.

The 6,11-dioxo-1,2,3,4,6,11-hexahydro-pyridazino[1,2-b]naphthalazine-1-carboxylic acid of Formula VIII that is used for resolution may be obtained by any process, including a process of this disclosure.

Suitable solvents that may be used in the reaction of step (a) include, but are not limited to: C₁₋₆ straight chain or branched alcohols, such as, for example, methanol, ethanol, isopropanol, butanol, and the like; ketones, such as, for example, acetone, ethyl methyl ketone, methyl isobutyl ketone, and the like; ethers, such as, for example, diethyl ether, dimethyl ether, diisopropyl ether, methyl tertiary-butyl ether, tetrahydrofuran, 1,4-dioxane, and the like; nitriles, such as, for example, acetonitrile, propionitrile, and the like; water; and any mixtures thereof.

Suitable optically pure amines include, but are not limited to, N-alkyl-D-glucamines and α-methylbenzylamine. Typical alkyl groups include methyl, ethyl, propyl, 2-propyl, cyclopropyl, butyl, 2-butyl, 11-dimethylethyl, cyclopropylmethyl, hexyl, cyclohexyl, octyl, dodecyl, octadecyl, and the like. For example, an optically pure amine may be N-octyl-D-glucamine.

Aspects of the invention include an optically pure n-alkyl-D-glucamine salt of 6,11-dioxo-1,2,3,4,6,11-hexahydro-pyridazino[1,2-b]naphthalazine-1-carboxylic acid.

Step (b) involves converting a diasteriomeric salt of Formula X into (S)-6,11-dioxo-1,2,3,4,6,11-hexahydro-pyridazino[1,2-b]naphthalazine-1-carboxylic acid or a salt thereof.

Suitable reagents for the conversion include, but are not limited to, strong bases. Suitable strong bases include, but are not limited to, aqueous alkali metal hydroxides, such as, for example, potassium hydroxide, sodium hydroxide, and the like.

The optically pure (S)-6,11-dioxo-1,2,3,4,6,11-hexahydro-pyridazino[1,2-b]naphthalazine-1-carboxylic acid may be converted into a salt or it may used directly in a succeeding reaction step.

Aspects of the invention include processes for racemizing (R)-6,11-dioxo-1,2,3,4,6,11-hexahydro-pyridazino[1,2-b]naphthalazine-1-carboxylic acid, embodiments comprising reacting (R)-6,11-dioxo-1,2,3,4,6,11-hexahydropyridazino[1,2-b]naphthalazine-1-carboxylic acid with a suitable reagent, to obtain 6,11-dioxo-1,2,3,4,6,11-hexahydro-pyridazino[1,2-b]naphthalazine-1-carboxylic acid.

Suitable reagents that may be used for the racemization reaction include, but are not limited to, acid anhydrides, bases, and mixtures thereof. Suitable acid anhydrides include, but are not limited to, acetic anhydride, propionic anhydride, butyric anhydride, benzoic anhydride, trifluoroacetic anhydride, and the like. Suitable bases include, but are not limited to, sodium or potassium acetate, sodium or potassium methoxide, sodium or potassium ethoxide, sodium or potassium *tert-*butoxide, and sodium or potassium carbonate.

The intermediate compounds described hereinabove, which are obtainable by the processes described hereinabove, may be used in the preparation of cilazapril according to, for example, processes known in the art.

Certain specific aspects and embodiments of the invention are described in the examples below, which are provided only for purposes of illustration and are not intended to limit the scope of the invention in any manner.

### EXAMPLES

### EXAMPLE 1: PREPARATION OF POTASSIUM SALT OF MALONIC ACID MONOMETHYL ESTER.

Diethyl malonate (500 g) and methanol (1 L) are placed into a round-bottom flask. The mass is cooled to about 10-15°C and stirred for about 30 minutes. A solution of 201 g of KOH in 1 L of methanol is added at about 10-15°C over about 6 hours. The mixture is stirred at 25-35°C for about 8 hours. The solvent is distilled under reduced pressure and the residue is cooled to 25-35°C. Ethyl acetate (250 mL) is added and the mixture is cooled to -5 to 5°C and stirred for about 4 hours. The formed solid is filtered and washed with 250 mL of chilled ethyl acetate. The wet product is dried in a vacuum oven at 60-65°C for about 10 hours, to afford the title compound. Approximate yield: 440 g. Assay by HPLC: 94.52%.

### EXAMPLE 2: PREPARATION OF 2,4-PENTADIENYLMETHYL ESTER USING PYRIDINE HCI.

N,N-Dimethylformamide (600 mL), malonic acid monomethyl ester potassium salt (300 g), pyridine HCI (233.2 g), and pyridine (7.6 g) are placed in a round-bottom flask equipped with a mechanical stirrer, under a nitrogen atmosphere. The mass is heated to 40-45°C and stirred under a nitrogen atmosphere for about 90 minutes. The mixture is cooled to 25-35°C and acrolein (161.2 g) is added at this temperature over about 50 minutes. The mixture is stirred at 25-35°C for about 25 hours and then cooled to 20-25°C. Water (3 L) is added and stirring is continued for about 30 minutes. Dichloromethane (1.5 L) is added, stirred, and separated from the aqueous layer. Additional dichloromethane (600 mL) is added, stirred, and separated from the aqueous layer. The organic layers are combined and washed with a mixture of 75 mL of concentrated HCl and 1.5 L of water. The aqueous layer is separated and extracted with dichloromethane (150 mL). The total organic layer is washed with water (1.5 L) to obtain the title compound in a dichloromethane solution. Approximate yield: 142.2 g.

### EXAMPLE 3: PREPARATION OF 6,11-DIOXO-1,4,6,11-TETRAHYDRO-PYRIDAZINO[1,2-B]PHTHALAZINE-1-CARBOXYLIC ACID METHYL ESTER.

Water (711 mL) and concentrated HCl (284 mL) are placed into a round-bottom flask. 2,3-Dihydrophthalazine-1,4-dione (288 g) is added at 25-35°C. The mass is cooled to -10 to 0°C and 2,4-pentadienylmethyl ester (142.2 g) in dichloromethane is added at the same temperature. Sodium hypochlorite solution (12% w/w, 1140 g) is added slowly over about 7 hours, below 0°C. The reaction is maintained for about 2 hours. A solution of 30 g of sodium bisulfite in 207 mL of water is added below 5°C, stirred for 15 minutes, and the organic layer is separated. The aqueous layer pH is adjusted to about 5 by adding a solution of 128 g of NaOH in 640 mL water at 0-5°C, and is stirred for 30 minutes. Undissolved solid is filtered and the solid is washed with dichloromethane (284 mL). The aqueous layer is separated from the filtrate and is washed with dichloromethane (284 mL). The combined organic layer is washed with water (711 mL) and the final organic layer is distilled. Acetone (426 mL) is added to the residue and the acetone is distilled. Acetone (284 mL) is charged to the reaction mixture and 142 mL is distilled. The residue is cooled to 0-5°C and stirred for about 2 hours. The formed solid is filtered and washed with chilled acetone (213 mL). The wet solid is dried under vacuum at 25-35°C for 30 minutes, and then at 50-55°C for 7 hours, to obtain the title compound. Approximate yield: 171 g. Purity by HPLC: 98.8%.

### EXAMPLE 4: PREPARATION OF 2,4-PENTADIENYLMETHYL ESTER USING PYRIDINE SULPHATE.

Dichloromethane (150 mL) and malonic acid monomethyl ester potassium salt (150 g ) are placed into a round-bottom flask equipped with a mechanical stirrer and cooled to 0-10°C. Sulfuric acid (51 g) is added slowly at 0-10°C. Dichloromethane (60 mL) and malonic acid monomethyl ester potassium salt (150 g) are added. Sulfuric acid (51 g) is added slowly at 0-10°C and stirred for 30 minutes. Pyridine (167 g) is added slowly, keeping the temperature at 5-20°C. Dimethylformamide (600 mL) is added and the mixture is heated to about 25-35°C. Acrolein (161.2 g) is added at this temperature over about 50 minutes. The mixture is stirred at 25-35°C for about 25 hours and then cooled to 20-25°C. Water (3 L) is added and stirring is continued for about 30 minutes. Dichloromethane (1290 mL) is added, stirred, and separated from the aqueous layer. Additional dichloromethane (600 mL) is added, stirred, and separated from the aqueous layer. The organic layers are combined and washed with mixture of 75 mL of concentrated HCl and 1.5 L of water. The aqueous layer is separated and extracted with dichloromethane (150 mL). The total organic layer is washed with water (1.5 L) to obtain the title compound as a solution in dichloromethane. Approximate yield: 142.2 g.

### EXAMPLE 5: PREPARATION OF 6,11-DIOXO-1,4,6,11-TETRAHYDRO-PYRIDAZINO[1,2-B]PHTHALAZINE-1-CARBOXYLIC ACID METHYL ESTER.

Water (711 mL) and concentrated HCl (284 mL) are placed in a round-bottom flask. 2,3-Dihydrophthalazine-1,4-dione (288 g) is added at 25-35°C. The mass is cooled to -10°C to 0°C and 2,4-pentadienylmethyl ester (142.2 g) in dichloromethane at -10°C to 0°C is added. Sodium hypochlorite solution (12% w/w, 1140 g) is added slowly over about 7 hours below 0°C, and is maintained for about 2 hours. A solution of 30 g of sodium bisulfite in 207 mL of water is added below 5°C, stirred for 15 minutes, and the organic layer is separated. The pH of the aqueous layer is adjusted to about 5 by adding a solution of 128 g of NaOH in 640 mL water at 0-5°C and the mixture is stirred for 30 minutes. The solid is removed by filtration and the wet cake is washed with dichloromethane (284 mL). The aqueous layer is separated from the filtrate and washed with dichloromethane (284 mL). The combined organic layers are washed with water (711 mL) and the organic layer is distilled. Acetone (426 mL) is added to the residue and 284 mL of acetone is distilled. Acetone (284 mL) is added to the mixture and 142 mL of solvent is distilled. The residual mass is cooled to 0-5°C and stirred for about 2 hours. The formed solid is filtered and washed with chilled acetone (213 mL). The wet solid is dried under vacuum at 25-35°C for 30 minutes and then at 50-55°C for 7 hours. Approximate yield: 192.4 g. Purity by HPLC: 98.8%.

### EXAMPLE 6: PREPARATION OF 6,11-DIOXO-1,2,3,4,6,11-HEXAHYDRO-PYRIDAZINO[1,2-B]NAPHTHALAZINE-1-CARBOXYLIC ACID.

6,11-Dioxo-1,4,6,11-tetrahydropyridazino[1,2-b]phthalazine-1-carboxylic acid methyl ester (150 g), Raney nickel (15 g, pre-washed with water and methanol) and methanol (1.2 L) are placed in an autoclave and hydrogenated at 60-75 psi and 55-60°C for about 10 hours. The mixture is cooled and filtered through a Celite bed at 45°C. The bed is washed with methanol (300 mL). Methanol is distilled from the filtrate below 55°C under vacuum. Water (450 mL) is added to the concentrated mass and distillation is continued to remove the methanol traces. The suspension is diluted with water (150 mL) and cooled to 25-35°C. A mixture of 33.7 g of sodium hydroxide in 112.5 mL of water is added at 25-40°C and stirred for about 2 hours at 65-75°C. The mixture is cooled to 45-55°C and pH is adjusted to about 7 using a mixture of 50 mL of sulphuric acid in 100 mL of water. Activated charcoal (7.5 g) is added and the mass is heated slowly to 60-65°C, and maintained for about 1 hour. The mass is filtered through a Celite bed and washed with hot water at 60-70°C. The pH of the filtrate is adjusted to 0.5 to 1 by adding dilute sulphuric acid solution at 40-45°C and the mixture is stirred for 1 hour. The mass is cooled to 20-25°C and maintained for about 2 hours. The formed solid is filtered and washed with water (450 mL). The wet solid is dried in a vacuum oven at 65-70°C for 8-10 hours to obtain the title product. Approximate yield: 126 g.

### EXAMPLE 7: RESOLUTION OF 6,11-DIOXO-1,2,3,4,6,11-HEXAHYDRO-PYRIDAZINO[1,2-B]NAPHTHALAZINE-1-CARBOXYLIC ACID.

6,11-Dioxo-1,2,3,4,6,11-hexahydropyridazino[1,2-b]naphthalazine-1-carboxylic acid (240 g), acetone (3420 mL), and water (180 mL) are charged into a round-bottom flask. The mixture is heated to 43°C and N-octyl-D-glucamine (131.5 g) is added and rinsed with a mixture of acetone (190 mL) and water (10 mL). The mixture is cooled to 41 °C, stirred for 5 hours at 41 °C, and further stirred at 15-20°C for 5 hours. The suspension is filtered and the solid is washed six times with 144 mL of a 95% aqueous acetone solution. The wet cake is dried at 60°C to obtain approximately 184 g of N-octyl-D-glucamine salt of (S)-6,11-dioxo-1,2,3,4,6,11-hexahydropyridazino[1,2-b]naphthalazine-1-carboxylic acid.

The salt (184 g) and water (2 L) are charged into a round-bottom flask equipped with a mechanical stirrer under a nitrogen atmosphere. The pH is adjusted to about 13 by slowly adding 50.3 mL of potassium hydroxide solution (48% w/w) over about 30 minutes. The suspension is stirred at 25°C for 4 hours, filtered, and rinsed six times with 97 mL of water at 25°C. The filtrate is transferred to a round-bottom flask and 38% aqueous HCl (122 mL) is added slowly at 15-30°C until the pH is less than 1. The suspension is stirred for 30 minutes at 25°C and cooled to 0-5°C. The solid is filtered and washed with water (6×58 mL). The wet solid is dried at about 60°C to obtain the title compound. Approximate yield: 84 g.

### EXAMPLE 8: RECOVERY OF N-OCTYL-D-GLUCAMINE AND (R)-6,11-DIOXO-1,2,3,4,6,11-HEXAHYDROPYRIDAZINO[1,2-B]NAPHTHALAZINE-1-CARBOXYLIC ACID.

The mother liquors obtained In Example 7 during the resolution of 6,11-dioxo-1,2,3,4,6,11-hexahydropyridazino[1,2-b]naphthalazine-1-carboxylic acid (4360 mL) are concentrated to 840 mL by distillation under vacuum. Aqueous KOH solution (48%, 32 mL) is added slowly to the mixture to obtain pH 7, at about 15-30°C. Water (500 mL) is added and the distillation is continued under vacuum to a volume of 840 mL. The solution is filtered to remove undissolved solids and the filtrate pH is adjusted to about 13 using aqueous KOH solution (48%, 16 mL). The resulting suspension is stirred at 25°C for 2 hours. The solid is filtered and washed with water (6x60 mL). The wet solid is dried to obtain approximately 30 g of N-octyl-D-glucamine as a solid.

The filtrate is charged into a flask and aqueous HCl (36%, 66 mL) is added over about 1 hour. The resulting suspension is stirred at 25°C for 30 minutes, cooled to 0-5°C, and stirred for about 2 hours. The solid is filtered and washed with water (6x100 mL). The solid is dried under vacuum below 60°C to obtain approximately 150 g of (R)-6,11-dioxo-1,2,3,4,6,11-hexahydropyridazino[1,2-b]naphthalazine-1-carboxylic acid, containing about 20% of the (S)-isomer.

### EXAMPLE 9: RACEMIZATION PROCESS.

(R)-6,11-dioxo-1,2,3,4,6,11-hexahydropyridazino[1,2-b]naphthalazine-1-carboxylic acid (150 g), acetic acid (270 mL), acetic anhydride (132 mL), and sodium acetate (47.5 g) are charged into a 1 L flask and heated to 70-75°C. The mixture is maintained for about 1 hour at 70-75°C and then transferred into another flask containing water (1115 mL) at 25-40°C. Aqueous H₂SO₄ solution (50%, 64.3 mL) is added at 25-35°C. The mixture is stirred for 2 hours at about 40°C, cooled to 0-5°C, and stirred for about 6 hours. The solid is filtered and washed with water (7x85.7 mL). The wet solid is dried under vacuum below 60°C to obtain racemic 6,11-dioxo-1,2,3,4,6,11-hexahydropyridazino[1,2-b]naphthalazine-1-carboxylic acid. Approximate yield: 139.5 g.

## Claims

1. A process for the preparation of 6,11-dioxo-1,2,3,4,6,11-hexahydropyridazino[1,2-b]naphthalazine-1-carboxylic acid or a salt or ester thereof, comprising at least one of the steps of:
(a) reacting a monoalkyl malonic acid of Formula II or a salt thereof with acrolein of Formula III, in the presence of a pyridine reagent, to obtain a 2,4-pentadienylalkyl ester of Formula IV, 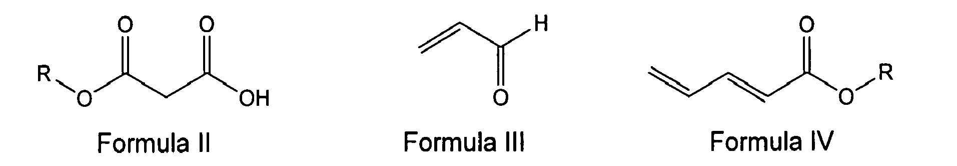 wherein R is C₁₋₆ alkyl, benzyl, or phenyl;
(b) reacting a 2,4-pentadienylalkyl ester of Formula IV with phthalizide of Formula V, in the presence of an oxidizing agent, to give a 6,11-dioxo-1,4,6,11-tetrahydropyridazino[1,2-b]phthalazine-1-carboxylic acid alkyl ester of Formula VI, 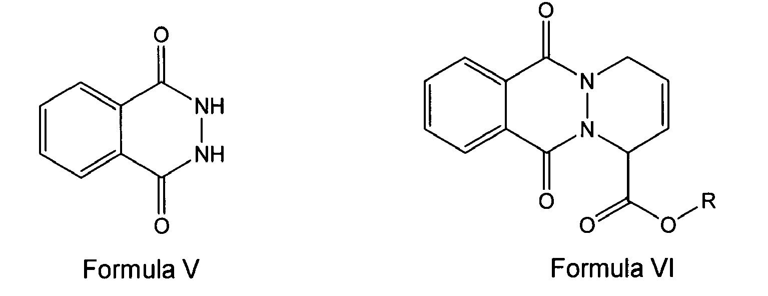 wherein R is as defined hereinabove; and
(c) reducing a 6,11-dioxo-1,4,6,11-tetrahydropyridazino[1,2-b]phthalazine-1-carboxylic acid alkyl ester of Formula VI to obtain an ester intermediate of Formula VII.

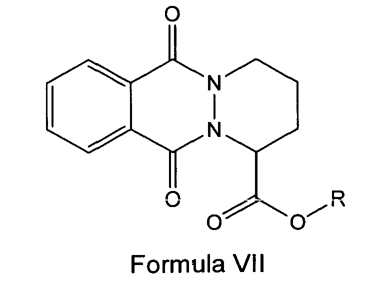

2. The process of claim 1, further comprising hydrolyzing an ester intermediate of Formula VII to give 6,11-dioxo-1,2,3,4,6,11-hexahydropyridazino[1,2-b]naphthalazine-1-carboxylic acid of Formula VIII.

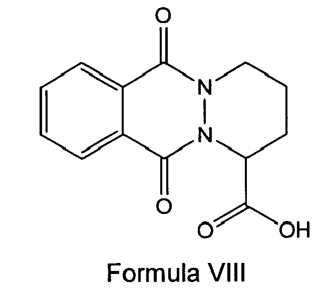

3. The process of claim 1, wherein a monoalkyl malonic acid of Formula II or a salt thereof is prepared by a process comprising reacting a dialkyl malonate with a base, in an alcohol solvent.

4. The process of claim 3, wherein a potassium salt of monomethyl malonate is prepared by reacting diethyl malonate with KOH in methanol.

5. The process of claim 1, wherein the reaction of step a) is carried out in the presence of an anhydrous acid.

6. The process of claim 1, wherein an oxidizing agent comprises sodium hypochlorite.

7. The process of claim 1, wherein reducing is carried out using a nickel-containing catalyst and hydrogen.

8. A process for the preparation of (S)-6,11-dioxo-1,2,3,4,6,11-hexahydropyridazino[1,2-b]naphthalazine-1-carboxylic acid of Formula IX, 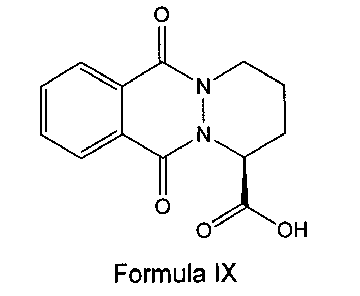 or a salt thereof, comprising at least one of the steps of:
(a) reacting 6,11-dioxo-1,2,3,4,6,11-hexahydropyridazino[1,2-b]naphthalazine-1-carboxylic acid of Formula VIII with an optically pure amine, to form a diastereomeric salt of Formula X, 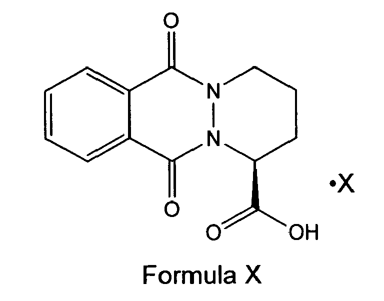 wherein X is an optically pure amine; and
(b) converting a diastereomeric salt of Formula X into (S)-6,11-dioxo-1,2,3,4,6,11-hexahydropyridazino[1,2-b]naphthalazine-1-carboxylic acid, or a salt thereof.

9. The process of claim 8, wherein an optically pure amine comprises N-octyl-D-glucamine.

10. The process of claim 8, wherein step a) is carried out in a solvent comprising a mixture of acetone and water.

11. A compound, which is an optically pure N-alkyl-D-glucamine salt of 6,11-dioxo-1,2,3,4,6,11-hexahydropyridazino[1,2-b]naphthalazine-l-carboxylic acid.

12. A process for racemizing (R)-6,11-dioxo-1,2,3,4,6,11-hexahydropyridazino[1,2-b]naphthalazine-1-carboxylic acid, comprising reacting (R)-6,11-dioxo-1,2,3,4,6,11-hexahydropyridazino[1,2-b]naphthalazine-1-carboxylic acid with a mixture of acetic acid, acetic anhydride and sodium acetate, to obtain 6,11-dioxo-1,2,3,4,6,11-hexahydropyridazino[1,2-b]naphthalazine-1-carboxylic acid.

13. A process for the preparation of cilazapril or a salt thereof, comprising converting 6,11-dioxo-1,2,3,4,6,11-hexahydropyridazino[1,2-b]naphthalazine-1-carboxylic acid, or a salt thereof, prepared according to the process of claim 1, into cilazapril or a salt thereof.

14. A process for the preparation of cilazapril or a salt thereof, comprising converting (S)-6,11-dioxo-1,2,3,4,6,11-hexahydropyridazino[1,2-b]naphthalazine-1-carboxylic acid prepared by the process of claim 8, or a salt thereof, into cilazapril or a salt thereof.

15. Use of optically pure N-alkyl-D-glucamine salts of 6,11-dioxo-1,2,3,4,6,11-hexahydro-pyridazino[1,2-b]naphthalazine-1-carboxylic acid in the preparation of cilazapril or a salt thereof.
